(19)

Europäisches
Patentamt
European
Patent Office
Office européen
des brevets

(11) **EP 4 579 279 A1**

(12) **EUROPEAN PATENT APPLICATION**

(43) Date of publication:
02.07.2025 Bulletin 2025/27

(21) Application number: 24223278.3

(22) Date of filing: 25.12.2024

(51) International Patent Classification (IPC):
*G01S 7/52* (2006.01)    *G01S 15/89* (2006.01)
*A61B 8/00* (2006.01)

(52) Cooperative Patent Classification (CPC):
**G01S 7/52046; A61B 8/5207; A61B 8/5269;
A61B 8/5276; G01S 7/52026; G01S 7/52049;
G01S 15/8979**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 26.12.2023  CN 202311814636

(71) Applicant: **Wuhan United Imaging Healthcare Co.,
Ltd.**
**WuHan, Hubei 430206 (CN)**

(72) Inventor: **LIU, Kaiwen**
**430206, Wuhan (CN)**

(74) Representative: **Wang, Bo**
**Panovision IP**
**Ebersberger Straße 3**
**85570 Markt Schwaben (DE)**

(54) **METHODS, SYSTEMS, AND STORAGE MEDIUM FOR DATA PROCESSING**

(57)    Embodiments of the present disclosure provide a method, system, and storage medium for data processing. The method comprises obtaining acquisition data of a target object, performing phase shift detection on the acquisition data to obtain phase shift information, performing phase correction to obtain correction data, and generating a medical image based on the correction data.

**300**

FIG. 3

EP 4 579 279 A1

**Description**

**CROSS-REFERENCE TO RELATED APPLICATIONS**

This application claims priority to Chinese Application No. 202311814636.5, filed on December 26, 2023, the entire contents of which are incorporated herein by reference.

**TECHNICAL FIELD**

The present disclosure relates to the field of data processing technology, and in particular, to a method, system, and storage medium for data correction.

**BACKGROUND**

In beamforming, persistence, and compounding techniques, signals from target structures of multiple elements, successive frames or different parameters are coherent, whereas noise is not. A plurality of acquisition channels can each acquire a plurality of data sets. The data sets are coherent with each other, while the noise within these data sets is incoherent. Therefore, by performing beamforming and/or medical image compositing on a plurality of data sets, the noise within these data sets can be eliminated, thereby improving the signal-to-noise ratio.

However, the acquisition times of the plurality of data sets associated with a target object collected by a plurality of acquisition channels are different, and the target object is prone to undergoing motion (e.g., heartbeat, respiration, etc.) during these various acquisition times, resulting in motion artifacts in the medical image generated based on the plurality of data sets corresponding to the acquisition times. In addition, different tissues exist within the target object, and the propagation speed of ultrasound waves and/or reflected ultrasound waves tend to vary and/or be uneven across different tissues, which can lead to inaccurate sound speed estimation used for beamforming, resulting in deviations in the delay processing of a plurality of channels and preventing the alignment of a plurality of data sets in time. Furthermore, delay mismatches between acquisition channels (e.g., mismatches in PCB trace length, cable length, transmission and/or reception device delays, transducer element mismatches, and mismatches in transducer matching layers and lenses) can also affect the alignment of data sets corresponding to the plurality of channels.

Therefore, there is a need for a data processing scheme that can align a plurality of data sets acquired at different times and/or by different acquisition channels so as to improve the signal-to-noise ratio of the data after beamforming and/or medical image compositing.

**SUMMARY**

According to an aspect of the present disclosure, a method for data processing is provided. The method may be implemented on at least one machine each of which has at least one processor and a storage device. The method may include obtaining acquisition data of a target object; obtaining phase shift information by performing phase shift detection on the acquisition data; and generating a target image by processing the acquisition data based on the phase shift information.

According to another aspect of the present disclosure, a system is provided. The system may include at least one storage medium storing a set of instructions and at least one processor configured to communicate with the at least one storage medium. When executing the set of instructions, the at least one processor may be directed to cause the system to perform operations including: obtaining acquisition data of a target object; obtaining phase shift information by performing phase shift detection on the acquisition data; and generating a target image by processing the acquisition data based on the phase shift information.

According to yet another aspect of the present disclosure, a non-transitory computer readable medium is provided. The non-transitory computer readable medium may include at least one set of instructions. When executed by at least one processor of a computer device, the at least one set of instructions may direct the at least one processor to perform operations including: obtaining acquisition data of a target object; obtaining phase shift information by performing phase shift detection on the acquisition data; and generating a target image by processing the acquisition data based on the phase shift information.

**BRIEF DESCRIPTION OF THE DRAWINGS**

The present disclosure will be further illustrated by way of exemplary embodiments, which will be described in detail by means of the accompanying drawings. These embodiments are not limiting, and in these embodiments, the same numbering denotes the same structure, where:

FIG. 1 is a schematic diagram illustrating an exemplary application scenario of a system for data processing according to some embodiments of the present disclosure;

FIG. 2 is a schematic diagram illustrating exemplary modules of a system for data processing according to some embodiments of the present disclosure;

FIG. 3 is a flowchart illustrating an exemplary process for data processing according to some embodiments of the present disclosure;

FIG. 4 is a flowchart illustrating an exemplary process for using a pattern matching algorithm to perform phase shift detection according to some embodiments of the present disclosure;

FIG. 5 is a flowchart illustrating an exemplary process for obtaining a bitwise cross-correlation data stream according to some embodiments of the present disclosure;

FIGs. 6A and 6B are schematic diagrams illustrating displacements of a target object at different acquisition times according to some embodiments of the present disclosure;

FIGs. 7A and 7B are schematic diagrams illustrating ideal states and actual states of alignment of acquisition data corresponding to different transducer channels according to some embodiments of the present disclosure;

FIG. 8 is a schematic diagram illustrating phase shift detection performed on a reference set and a target set based on a matching window and a searching window according to some embodiments of the present disclosure;

FIG. 9 is a schematic diagram illustrating an exemplary phase shift event bit stream corresponding to acquisition data according to some embodiments of the present disclosure;

FIG. 10 is a schematic diagram illustrating two exemplary manners of converting acquisition data into a phase shift event bit stream according to some embodiments of the present disclosure;

FIGs. 11A and 11B are schematic diagrams illustrating acquisition data and a corresponding phase shift event bit stream according to some embodiments of the present disclosure;

FIG. 12 is a schematic diagram illustrating an operating principle of phase shift detection performed on a phase shift event bit stream according to some embodiments of the present disclosure; and

FIG. 13 is a schematic diagram illustrating an exemplary user interaction interface according to some embodiments of the present disclosure.

## DETAILED DESCRIPTION

In order to more clearly illustrate the technical solutions of the embodiments of the present disclosure, a brief description of the accompanying drawings required to be used in the description of the embodiments is given below. Obviously, the accompanying drawings in the following description are only some examples or embodiments of the present disclosure, and it is possible for a person of ordinary skill in the art to apply the present disclosure to other similar scenarios in accordance with these drawings without creative labor. Unless obviously obtained from the context or the context illustrates otherwise, the same numeral in the drawings refers to the same structure or operation.

It should be understood that the terms "system", "device", "unit" and/or "module" as used herein is a way to distinguish between different components, elements, parts, sections, or assemblies at different levels. However, other expressions may replace words if other words accomplish the same purpose.

As shown in the present disclosure and in the claims, unless the context clearly suggests an exception, the words "one", "a", "an," "one kind," and/or "the" do not refer specifically to the singular, but may also include the plural. Generally, the terms "including" and "comprising" suggest only the inclusion of clearly identified steps and elements that do not constitute an exclusive list, and the method or apparatus may also include other steps or elements.

Flowcharts are used in the present disclosure to illustrate operations performed by a system according to embodiments of the present disclosure. It should be appreciated that the preceding or following operations are not necessarily performed in an exact sequence. Instead, steps can be processed in reverse order or simultaneously. Also, it is possible to add other operations to these processes, or to remove a step or steps from these processes.

Beamforming is a key technology used in many sensor array systems such as radar, sonar, medical ultrasound imaging systems, and audio array systems. It also plays a key role in wireless communication including 5G, LTE and WLAN. The benefits of beamforming are to focus the transmitted energy to the focal locations while doing transmit beamforming, and to focus the received signals and improve the signal-to-noise (SNR) ratio of received signals. The focusing of the receive signals is achieved by realignment of signals from multiple receive channels and then do coherently summation. The realignment of signals can be achieved by delaying or phase rotation. Windowing can be used by applying different weights to different channels before summation. Conventional beamformers use predefined weights. More advanced adaptive beamformers have variable weights based on the analysis result of the signals, for example minimal variance.

In ultrasound systems, persistence is a temporal filter used in both 2D gray scale imaging and color Doppler imaging when successive frames are averaged with or without windowing to improve the SNR of the images and make the images smoother. The windowing can be represented by infinite impulse response (IIR) or finite impulse response (FIR) filters. Compounding is another way to achieve the same goal by imaging the same target object with different scan parameters and averaging the frames. For example, spatial compounding is a very common used compounding technique when the target object is imaged at various angles, the image frames of different angles are averaged to generate the compound ultrasound image. Frequency compounding uses filters of different cutoff frequencies to filter the signals and average the filter signals. When using compounding technology, the averaging of the frames can involve different windows or weighting.

In beamforming, persistence and compounding techniques, the signals from the structures of the target object from multiple elements, successive frames or different parameters are coherent and the noise is not. The averaging can then improve the image quality by improving the SNR. It's known that the SNR is the ratio of signal power and noise power. When averaging is applied, the improvement of SNR is proportional to the number of channels or frames involved in the averaging. Moreover, some structures can be more visible at certain angles. All these benefits make beamforming, persistence and spatial compounding very popular in ultrasound imaging systems.

However, because both persistence and compounding techniques involve multiple frames acquired in different times, any motion between the frames can cause artifacts and make the image blurring and have adverse effect on the image quality. Tissue movement such as heart beat can cause motion, the operators can also move the transducer intentional or unintentional. The faster the movement, the worse it can be. Another factor is the estimation accuracy of speed of sound. When the speed of sound used for beamforming is different from the actual speed of sound in the tissue, the image quality is affected. To make the problem more complex, the accurate estimation of speed of sound is impossible or pointless because different types of tissues have different speeds of sound (tissue inhomogeneity). In a typical ultrasound system, the speed of sound in soft tissue, which is 1540 m/s is often used to calculate the delay profiles for beamforming. When scanning different types of tissues or when there are different types of tissues in the region of interest, the accuracy of beamforming become questionable. Yet another factor is the delay mismatch between channels. Such as printed circuit board (PCB) trace length mismatch, cables length mismatch, the ultrasound transmitting and/or receiving devices (integrated circuits in most cases) delay mismatch, the transducer element to element mismatch, transducer matching layer and lenses mismatch, etc. A further factor is errors from the calculation or estimation of wave travel time used in most beamforming algorithms.

The present application uses the phase information from the signals to fine tune the delay mismatch errors from beamforming, coherent persistence, coherent compounding or any other related signal processing techniques. The tuning process can precisely realign the data from different channels within acquisitions and improve the performance of beamforming. The tuning process can also realign data from multiple acquisitions or frames during coherent persistence and compounding to correct the mis-registration from motion errors and steer angle errors between frames. With this method, super high resolution ultrasound image can be achieved in real time. Furthermore, this method can also be used to measure the velocity of the tissue movement or blood flow at the same time producing high definition, high frame rate 2D or 3D images.

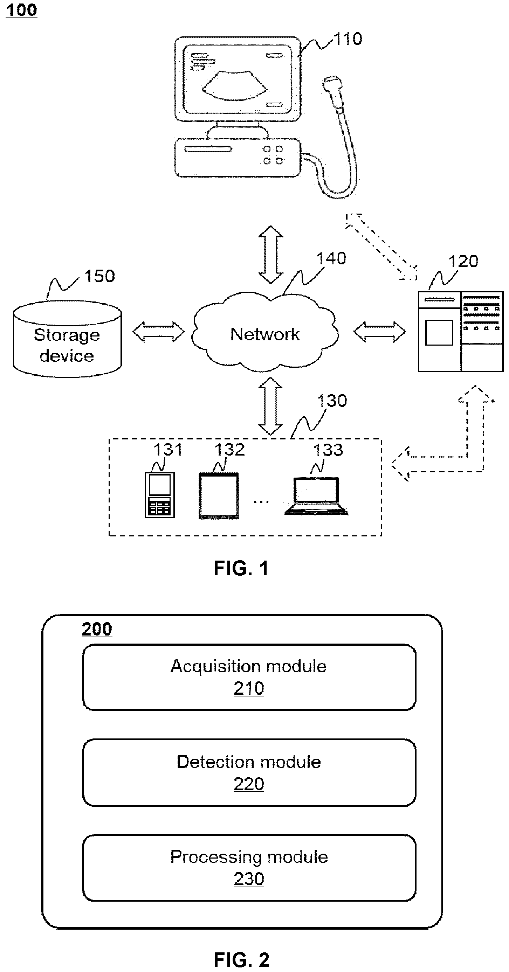

FIG. 1 is a schematic diagram illustrating an exemplary application scenario of a system for data processing according to some embodiments of the present disclosure. A system 100 for data processing may implement the methods and/or processes disclosed herein to align a plurality if data sets acquired at different times or by different acquisition channels, thereby enhancing a signal-to-noise ratio of data after beamforming and/or image compositing. As shown in FIG. 1, the system 100 for data processing may include an imaging device 110, a processing device 120, a terminal device 130, a network 140, and/or a storage device 150, etc.

Components of the system 100 for data processing may be connected in one or more various ways. For illustration purpose only, the imaging device 110 may be connected to the processing device 120 via the network 140, as shown in FIG. 1. As another example, the imaging device 110 may be directly connected to the processing device 120 (as shown by a dashed bidirectional arrow connecting the imaging device 110 and the processing device 120). As a further example, the storage device 150 may be connected to the processing device 120 either directly or via the network 140. As a further example, the terminal device 130 may be connected to the processing device 120 directly (as indicated by a dashed bidirectional arrow connecting the terminal device 130 and the processing device 120) and/or via the network 140.

The imaging device 110 may scan a target object to obtain acquisition data. In some embodiments, the imaging device 110 emits signal(s) (e.g., ultrasound waves) to the target object or a portion of the target object and receives reflected signal(s) (e.g., reflected ultrasound waves) from the target object or a portion of the object. In some embodiments, an imaging device includes but is not limited to, a computed tomography (CT) system, a computed tomography angiography (CTA) system, a positron emission tomography (PET) system, a single photon emission computed tomography (SPECT) system, a magnetic resonance imaging (MRI) system, a digital subtraction angiography (DSA) system, an ultrasound scanning (US) systems or thermal tomography (TTM) systems, etc. For ease of illustration, the present disclosure is described for the purpose of illustration in which the imaging device is an ultrasound scanning system and the acquisition data is ultrasound imaging data.

The processing device 120 may process data and/or information obtained from the imaging device 110, the terminal device 130, and/or the storage device 150. For example, the processing device 120 performs phase shift detection on the acquisition data to obtain phase shift information. As another example, the processing device 120 processes the acquisition data based on the phase shift information to obtain a target image. As another example, the processing device 120 performs phase correction on the acquisition data based on the phase shift information to obtain correction data. In some embodiments, the processing device 120 includes a central processing unit (CPU), a digital signal processor (DSP), a system on a chip (SoC), a microcontroller unit (MCU), etc., and/or any combination thereof. In some embodiments, the processing device 120 includes a computer, a user console, a single server, a set of servers, or the like. The set of servers may be centralized or distributed. In some embodiments, the processing device 120 is local or remote. For example, the processing device 120 may access information and/or data stored in the imaging device 110, the terminal device 130, and/or the storage device 150 via the network 140. As another example, the processing device 120 may directly connect to the imaging device 110, the terminal device 130, and/or the storage device 150 to access stored information and/or data. In some embodiments, the processing device 120 is implemented on a cloud platform. Merely by way of example, a cloud platform includes a private cloud, a public cloud, a hybrid cloud, a community cloud, a distributed cloud, an inter-cloud, a multi-cloud, etc., or any combination thereof. In some embodiments, the processing device 120 or a portion of the processing device 120 is integrated into the imaging device 110.

The terminal device 130 may display a medical image to a user. The terminal device 130 may include a mobile device 131, a tablet computer 132, a laptop computer 133, etc., or any combination thereof. In some embodiments, the terminal device 130 may be a portion of the processing device 120.

The network 140 may include any suitable network that facilitates the exchange of information and/or data of the system 100 for data processing. In some embodiments, one or more components of the system 100 for data processing (e.g., the imaging device 110, the processing device 120, the storage device 150, the terminal device 130) communicate information and/or data with one or more other components of the system 100 for data processing via the network 140. For example, the processing device 120 may send the medical image to an end device via the network 140. As another example, the processing device 120 may obtain the acquisition data from the imaging device 110 via the network 140. The network 140 may be and/or include a public network (e.g., the Internet), a private network (e.g., a local area network (LAN), a wide area network (WAN)), a wired network (e.g., Ethernet network), a wireless network (e.g., an 802.11 network, a Wi-Fi network), a cellular network (e.g., a Long Term Evolution (LTE) network), a frame relay network, a virtual private network ( "VPN"), satellite networks, telephone networks, routers, hubs, switches, server computers, and/or any combination thereof. By way of example only, the network 140 includes a cable network, a wired network, a fiber optic network, a telecommunication network, an intranet, a wireless local area network (WLAN), a metropolitan area network (MAN), a public switched telephone network (PSTN), a Bluetooth™ network, a PurpleBee™ network, a near-field communication (NFC) network, or the like, or any combination thereof. In some embodiments, the network 140 includes one or more network access points. For example, the network 140 includes wired and/or wireless network access points, such as a base station and/or an Internet exchange point, through which one or more components of the imaging device 110 may connect to the network 140 to exchange data and/or information.

The storage device 150 may store data, instructions, and/or any other information. In some embodiments, the storage device 150 may store data obtained from the imaging device 110, the terminal device 130, and/or the processing device 120. In some embodiments, the storage device 150 may store data and/or instructions, and the processing device 120 may execute or use the data and instructions to execute exemplary methods/systems described herein. In some embodiments, the storage device 150 may include mass storage, removable memory, volatile read-write memory, read-only memory (ROM), or the like, or any combination thereof. Exemplary mass storage may include magnetic disks, optical disks, solid-state disks, or the like. Exemplary removable memory may include flash drives, floppy disks, optical disks, memory cards, zip disks, magnetic tapes, or the like. Exemplary volatile read-write memory may include random access memory (RAM). Exemplary RAMs may include Dynamic Random Access Memory (DRAM), Double Data Rate Synchronous Dynamic Random Access Memory (DDR SDRAM), Static Random Access Memory (SRAM), Thyristor Random Access Memory (T-RAM), and Zero Capacitance Random Access Memory (Z-RAM). Exemplary ROMs may include masked read-only memory (MROM), programmable read-only memory (PROM), erasable programmable read-only memory (EPROM), electrically erasable programmable read-only memory (EEPROM), compact disc read-only memory (CD-ROM), and digital multifunction disk read-only memory. In some embodiments, the storage device 150 is performed on a cloud platform. By way of example only, the cloud platform may include a private cloud, a public cloud, a hybrid cloud, a community cloud, a distributed cloud, an on-premises cloud, a multi-tiered cloud, etc., or any combination thereof.

It should be noted that the foregoing description is provided for illustrative purposes only and is not intended to limit the scope of the present disclosure. For a person of ordinary skill in the art, a wide variety of variations and modifications may be made under the guidance of the contents of the present disclosure. Features, structures, methods, and other characteristics of the exemplary embodiments described herein may be combined in various ways to obtain additional and/or alternative exemplary embodiments. However, these changes and modifications do not depart from the scope of the present disclosure.

FIG. 2 is a schematic diagram illustrating exemplary modules of a system for data processing according to some embodiments of the present disclosure. In some embodiments, a system 200 for data processing includes an acquisition module 210, a detection module 220, and/or a processing module 230.

The acquisition module 210 may be used to obtain acquisition data of a target object. A detailed description of the acquisition module 210 can be found in 310. In some embodiments, the acquisition data includes ultrasound imaging data. In some embodiments, the acquisition data includes a plurality of data sets. In some embodiments, the plurality of data sets include a reference set and a target set.

The detection module 220 may be configured to perform phase shift detection on the acquisition data to obtain phase shift information. In some embodiments, the detection module 220 performs one or more of the following operations: determining a matching window and a searching window; obtaining the phase shift information by determining, using a pattern matching algorithm based on the matching window and the searching window, one or more phase shift coefficients between the at least one target set and the reference set. In some embodiments, the one or more phase shift coefficients include at least one of a normalized cross correlation coefficient, a sum of square difference coefficient, or a sum of absolute differences coefficient. In some embodiments, the one or more phase shift coefficients include at least one bitwise cross-correlation data stream between the at least one target set and the reference set. In some embodiments, the detection module 220 performs one or more of the following operations: converting the acquisition data into a minimal quantized signal; and performing the phase shift detection on the minimal quantized signal to obtain a bitwise cross-correlation data stream of each channel between the at least one target set and the reference set. A detailed description of the detection module 220 can be found in 320.

The processing module 230 may be configured to process the acquisition data based on the phase shift information to generate a target image. In some embodiments, the processing module 230 is configured to obtain correction data by performing phase correction on the acquisition data based on the phase shift information. In some embodiments, the processing module 230 performs one or more of the following operations: performing, based on the phase shift information, phase alignment on inter-frame data among the acquisition data acquired at different times; and/or performing, based on the phase shift information, phase alignment on data of different channels among the acquisition data acquired at a same time. In some embodiments, the processing module 230 is configured to generate a medical image based on the correction data. In some embodiments, the processing module 230 performs one or more of the following operations: generating a composite image by performing multi-frame beamforming based on the correction data; and/or generating the target image by performing single-frame image reconstruction based on the correction data. A detailed description of the processing module 230 can be found in 330.

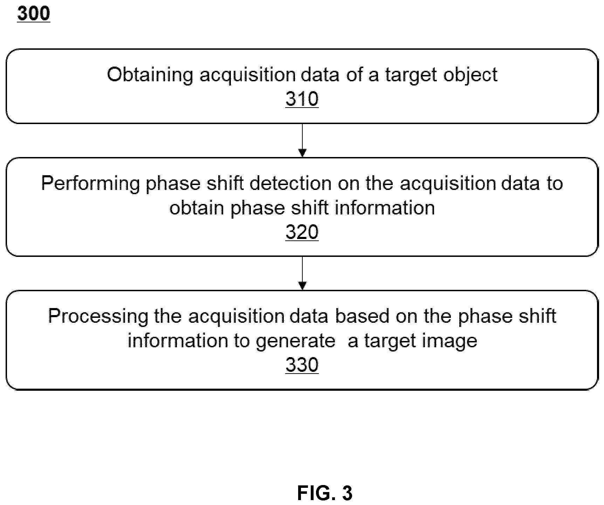

In some embodiments, the system 100 for data processing further comprises a determination module. The calculation module may be configured to determine a movement speed of a region of interest in the target object based on the phase shift information. A detailed description of the determination module can be found in FIG. 3.

It is to be noted that the above description of the system for data processing and its modules is for descriptive convenience only, and does not limit the present disclosure to the scope of the cited embodiments. It is to be understood that for a person skilled in the art, after understanding the principle of the device, it may be possible to arbitrarily combine the individual modules, or constitute a sub-unit to be connected to the others, without departing from this principle. In some embodiments, modules disclosed in FIG. 2 may be implemented on the same or different processing devices. In some embodiments, modules disclosed in FIG. 2 may be different modules in a single system, or a single module that implements the functionality of two or more of the modules described above. For example, individual modules share a common storage module, and the individual modules each have their own storage module. However, those variations and modifications do not depart the scope of the present disclosure.

FIG. 3 is a flowchart illustrating an exemplary method for data processing according to some embodiments of the present disclosure. In some embodiments, a process 300 is performed by the system 100 for data processing (e.g., the processing device 120) or the system 200 for data processing. For example, the process 300 is stored in a storage device in the form of a program or instructions, and the process 300 is implemented when the system 100 for data processing (e.g., the processing device 120) or the system 200 for data processing executes the instructions. The schematic diagram of operations of the process 300 presented below is illustrative. In some embodiments, one or more additional operations not described and/or one or more operations not discussed may be utilized to complete the process. Additionally, an order of the operations of the process 300 illustrated in FIG. 3 and described below is not limiting. As illustrated in FIG. 3, the process 300 may include one or more of the following operations.

In 310, acquisition data of a target object may be obtained. Specifically, the operation 310 is performed by the acquisition module 210.

The target object may be a human body, an organ, an organism, an object, an injury site, a tumor, and so on. The acquisition data may be medical data captured by an imaging device. For the purpose of ease of illustration, an example is illustrated with an ultrasound scanning system as the imaging device and ultrasound imaging data as the acquisition data. Specifically, an ultrasound probe may convert an electrical signal to an ultrasound signal through a transducer array element and transmit the ultrasound signal to the target object or a portion of the target object, and then receive reflected ultrasound waves from the target object or a portion of the target object and convert a reflected ultrasound signal to an electrical signal (i.e., the ultrasound imaging data) through the transducer array element. In some embodiments, the acquisition data includes a plurality of data sets.

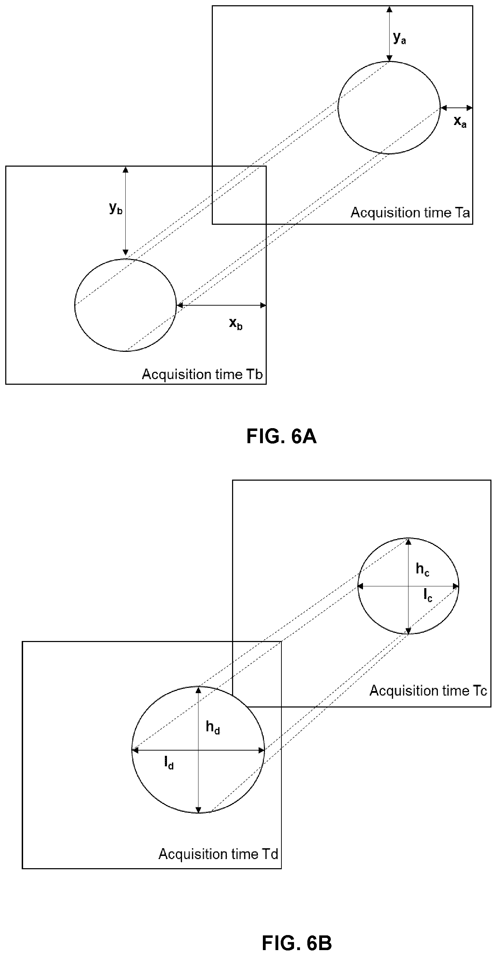

In some embodiments, each data set corresponds to a period of acquisition time. For example, the ultrasound scanning system acquires a data set a, a data set b, a data set c, and a data set d of the target object at acquisition times Ta, Tb, Tc, and Td, respectively. In some embodiments, the target object changes in position and/or shape at different acquisition times due to motion, resulting in misalignment of corresponding data sets (e.g., inconsistent phase information between data sets). FIGs. 6A and 6B are schematic diagrams illustrating displacements of a target object at different acquisition times according to some embodiments of the present disclosure. For example, as shown in FIG. 6A, the target object corresponds to a coordinate position of $(x_a, y_a)$ at the acquisition time Ta and a coordinate position of $(x_b, y_b)$ at the acquisition time Tb, and the change in position produced by the target object may result in a misalignment between a corresponding data set a and a corresponding data set b. As another example, as shown in FIG. 6B, the target object has a length $l_c$ and a height $h_c$ at the acquisition time Tc, and a length $l_d$ and a height $h_d$ at the acquisition time Td, and a change in the shape produced by the target object may result in the misalignment between a corresponding data set c and a corresponding data set d.

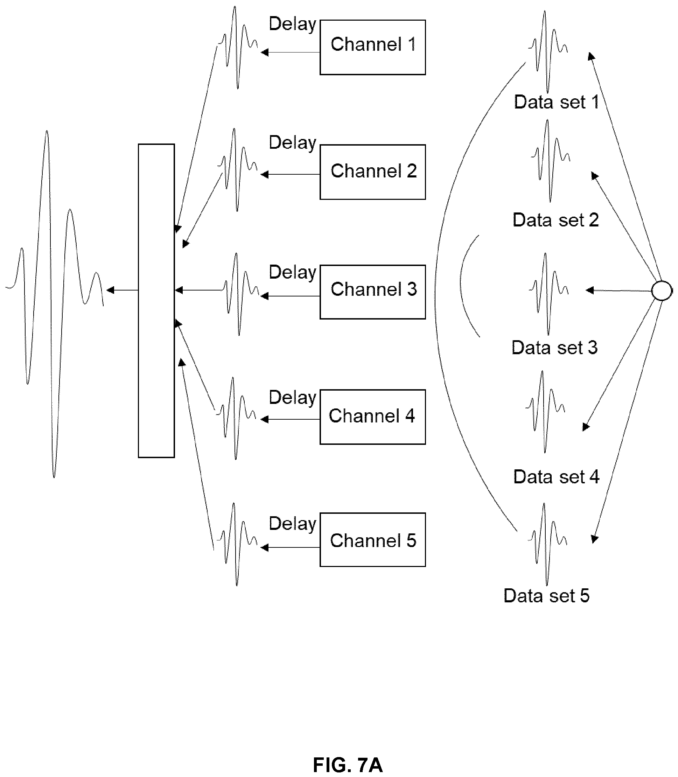

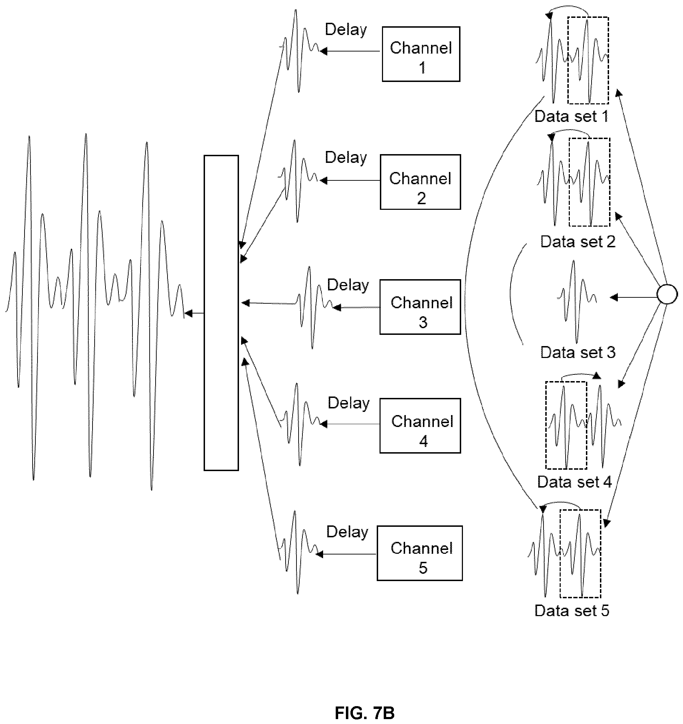

In some embodiments, each data set corresponds to an acquisition channel. For example, FIGs. 7A and 7B are schematic diagrams illustrating ideal states and actual states of alignment of acquisition data corresponding to different transducer channels according to some embodiments of the present disclosure. As shown in FIGs. 7A and 7B, transducer channels 1, 2, 3, 4, and 5 of the ultrasound probe may acquire data sets 1, 2, 3, 4, and 5, respectively. In some embodiments, distances between the target object and different transducer arrays are different, resulting in different reception times for data sets received by different transducer channels. Therefore, based on dimensional parameters of the ultrasound probe, delay processing may be applied to a plurality of data sets received by different transducer channels to achieve temporal alignment of the data sets. For example, as shown in FIG. 7A, in an ideal state, after applying the delay processing to a plurality of data sets, the data sets may be temporally aligned. Beamforming and/or compounding of the aligned data sets can then enhance and/or amplify a signal of the acquisition data. However, different tissues (e.g., water, fat, muscle, etc.) may be present in the target object, a propagation speed of ultrasound waves and/or reflected ultrasound waves may be different in different tissues of the target object, and a distribution of the different tissues may be non-uniform, resulting in biased delayed processing, and the plurality of data sets may not be temporally aligned. For example, as shown in FIG. 7B, in an actual state, after applying the delay processing to the plurality of data sets, the plurality of data sets may be not fully temporally aligned, and the signal enhancement may be limited and noise(s) may exist.

As can be seen from the foregoing, there may be a misalignment between a plurality of data sets corresponding to a plurality of acquisition times and/or a plurality of data sets corresponding to a plurality of acquisition channels, and thus alignment (or correction) between the plurality of data sets is required. In some embodiments, the plurality of data sets include a reference set and a target set. The reference set may be a data set that is used as a reference (or standard) in an alignment operation, and the target set may be a data set that is to be aligned based on the reference set. For example, as shown in FIGs. 6A and 6B, the data set a corresponding to the acquisition time Ta is the reference set, and the data sets b, c, and d corresponding to the acquisition times Tb, Tc, and Td are target sets. As another example, as shown in FIG. 7B, a data set 3 corresponding to a channel 3 is the reference set, and data sets 1, 2, 4, and 5 corresponding to channels 1, 2, 4, and 5 are the target sets.

In 320, phase shift detection may be performed on the acquisition data to obtain phase shift information. Specifically, the operation 320 may be performed by the detection module 220.

The phase shift information may describe information about phase shifts between the plurality of data sets. In some embodiments, the phase shift information includes a phase shift between the target set and the reference set. In some embodiments, the phase shift information is represented by a phase shift map. The phase shift map may include phase shift information between a plurality of positions in the reference set and the target set. For example only, the detection module 220 designates coordinates of a plurality of positions in the reference set as coordinates of a position with a phase shift of 0, and coordinates of a plurality of positions corresponding to the target set as a displacement with respect to a corresponding position in the reference set. For example, taking a two-dimensional data set corresponding to an acquisition time as an example, a phase shift of a position 1 of a target object in the reference set is (0, 0), and a phase shift of the position 1 in the target set is 2 along an x-direction and 3 along a y-direction, then coordinates of the position 1 in the phase shift map is expressed as (2, 3).

The phase shift detection may be the process of detecting the phase shift information.

In some embodiments, the processing module 230 utilizes a first machine learning model to perform the phase shift detection on the acquisition data to obtain the phase shift information. In some embodiments, the first machine learning model includes but is not limited to, a support vector machine model, a decision tree model, a random forest model, a convolutional neural network (CNN) model, a recurrent neural network (RNN), or the like. Specifically, an input to the first machine learning model is the acquisition data and an output of the first machine learning model is the phase shift information. The first machine learning model may extract a feature (e.g., a time-domain feature, a frequency-domain feature, a time-frequency-domain feature, etc.) of the acquisition data, and then output the phase shift information based on the feature of the acquisition data.

In some embodiments, the first machine learning model is obtained by training based on a plurality of first training samples with first labels. Specifically, the plurality of first training samples with the first label are input into an initial first machine learning model, and parameters of the initial first machine learning model are updated by training to obtain a trained first machine learning model. The first training samples of the first machine learning model include sample acquisition data, and the first labels include sample phase shift information. The sample acquisition data may be historical medical data acquired by the imaging device, and the sample phase shift information may be determined manually based on the sample acquisition data, or may be obtained by an algorithm (e.g., a pattern matching algorithm) or other manners with a relatively high degree of accuracy.

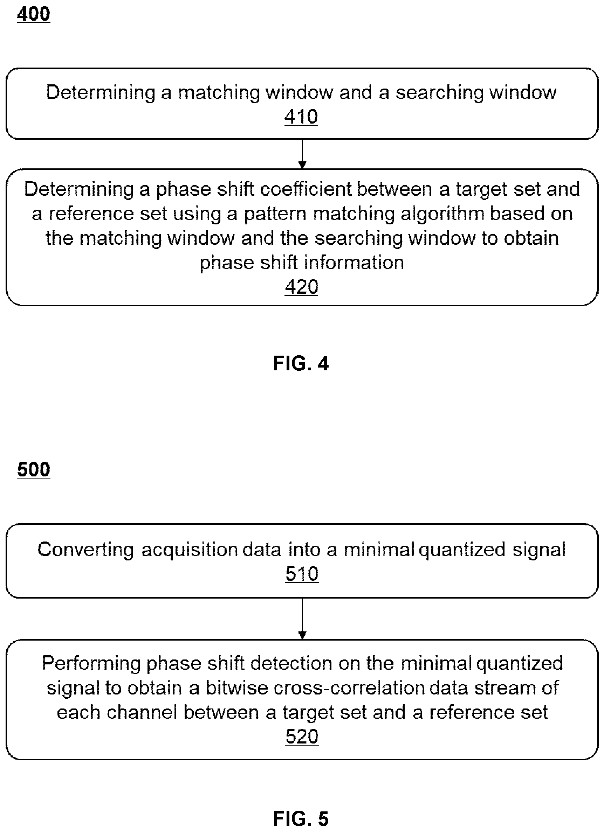

In some embodiments, the processing module 230 utilizes a pattern matching algorithm to perform the phase shift detection on the acquisition data to obtain the phase shift information. A detailed description of the phase shift detection using the pattern matching algorithm can be found in FIG. 4 and will not be repeated herein.

In some embodiments, a user selects to trigger the phase shift detection to obtain the phase shift information through a phase shift detection function interface on a user interaction interface. The phase shift detection function interface may include, but is not limited to, one or more of buttons, input boxes, selection boxes, sliders, menus, or the like, or any combination thereof.

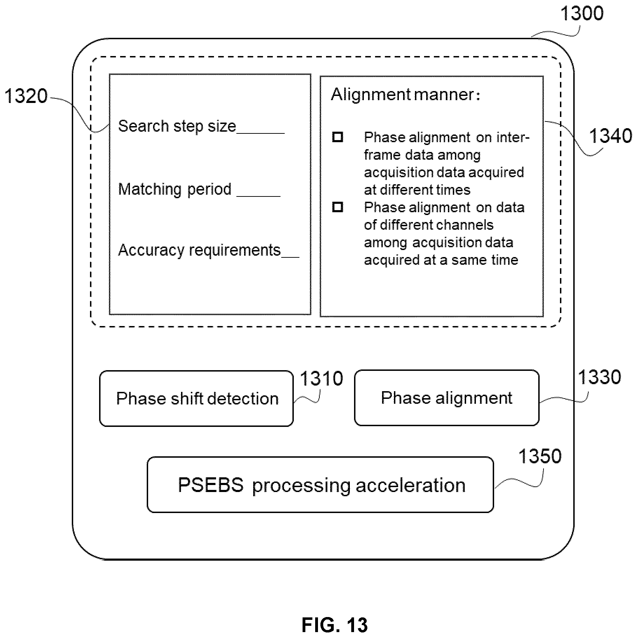

Specifically, the detection module 220 obtains a first user request. In some embodiments, the first user request indicates a user selects to trigger the phase shift detection to obtain the phase shift information through a phase shift detection function interface on a user interface. FIG. 13 is a schematic diagram illustrating an exemplary user interaction interface according to some embodiments of the present disclosure. As shown in FIG. 13, a user may trigger phase shift detection by clicking a phase shift detection button 1310 (i.e., a phase shift detection function interface) on a user interaction interface 1300. In some embodiments, the first user request indicates a user selects to trigger the phase shift detection to set one or more parameters of the phase shift detection through a phase shift detection function interface on a user interface. For example, as shown in FIG. 13, the user sets one or more parameters (e.g., accuracy requirements, search step size, matching period) of the phase shift detection function through a menu 1320 (i.e., the phase shift detection function interface) on the user interaction interface 1300. A detailed description of the accuracy requirements, the search step size, and the matching period can be found in FIG. 4 and its related descriptions. Furthermore, in response to the first user request, the detection module 220 performs the phase shift detection on the acquisition data.

In 330, the acquisition data may be processed based on the phase shift information to obtain a target image. Specifically, the operation 330 may be performed by the processing module 230.

Specifically, in some embodiments, the processing module 230 performs phase correction on the acquisition data based on the phase shift information to obtain correction data. The phase correction may be a process of aligning a phase of a target set with a phase of a reference set. The correction data may be data obtained after phase alignment is performed on the acquisition data. In some embodiments, the processing module 230 obtains a corrected target set and obtains the correction data by superimposing a target set and a corresponding phase shift map.

In some embodiments, the processing module 230 performs phase alignment on inter-frame data among acquisition data acquired at different times based on the phase shift information. Merely by way of example, a data set a corresponding to an acquisition time a is a reference set, and a data set b corresponding to an acquisition time b is a target set, and the processing module 230 obtains, based on the data set a and the data set b, a phase shift map b corresponding to a data set b, and obtains a corrected data set b by superimposing the data set b and the phase shift map b. Similarly thereto, the processing module 230 may obtain a phase shift map c corresponding to the data set c based on the data set a and the data set c corresponding to the acquisition time c, superimpose the data set c and the phase shift map c to obtain the corrected data set c; ...; until all corrected target sets are obtained.

In some embodiments, the processing module 230 performs the phase alignment on data of different channels among acquisition data acquired at a same time based on the phase shift information. Merely by way of example, a data set 1 corresponding to a channel 1 is a reference set, and a data set 2 corresponding to a channel 2 is a target set, and then a phase shift map 2 corresponding to the data set 2 is obtained based on the data set 1 and the data set 2, and a corrected data set 2 is obtained by superimposing the data set 2 and the phase shift map 2. Similarly, a phase shift map 3 corresponding to the data set 3 may be obtained based on the data set 1 and a data set 3 corresponding to a channel 3, and a corrected data set 3 may be obtained by superimposing the data set 3 and the phase shift map 3; the operations may be repeated until all corrected target sets are obtained.

In some embodiments of the present disclosure, performing the phase alignment on the inter-frame data among the acquisition data acquired at different times based on the phase shift information can eliminate motion artifacts of images generated from a plurality of data sets corresponding to a plurality of acquisition times; and/or by performing the phase alignment on data of different channels among the acquisition data acquired at a same time can eliminate deviations generated by applying delay processing to a plurality of channels or delay mismatches between acquisition channels.

Further, in some embodiments, the processing module 230 generates a medical image based on the correction data. Merely by way of example, the medical image is an ultrasound image. In some embodiments, formats for the medical image include, but are not limited to, Joint Photographic Experts Set (JPEG) format, Tagged Image File Format (TIFF) format, Graphics Interchange Format (GIF) format, Kodak Flash PiX (FPX) format, Digital Imaging and Communications in Medicine (DICOM) format, or the like. In some embodiments, the medical image is a two-dimensional (2D) image or a three-dimensional (3D) image.

In some embodiments, the processing module 230 performs multi-frame beamforming based on the correction data to generate a composite image. Specifically, the processing module 230 may composite a plurality of data sets corresponding to a plurality of channels of a plurality of corrected channels to obtain composite data and generate the composite image based on the composite data.

In some embodiments, the processing module 230 generates a target image by performing single-frame image reconstruction based on the correction data. Specifically, the processing module 230 generates a single-frame image based on a data set corresponding to each corrected acquisition time and then superimposes a plurality of single-frame images to generate the target image.

In some embodiments of the present disclosure, performing the multi-frame beamforming based on the correction data to generate the composite image can eliminate deviations generated by applying delay processing to a plurality of channels or delay mismatches between acquisition channels; and/or performing the single-frame image reconstruction based on the correction data to generate the target image can eliminate motion artifacts in a medical image generated from a plurality of data sets corresponding to acquisition data acquired at different times.

In some embodiments, by performing the phase shift detection on the acquisition data to obtain the phase shift information, and performing the phase correction on the acquisition data based on the phase shift information to obtain the correction data, such that the correction data can eliminate the effect of motion displacement between data sets corresponding to different frames (i.e., different acquisition times), the effect of delay processing due to different acquisition channels, and/or the effect of delay mismatches between acquisition channels and the system, and thus the signal-to-noise ratio of the medical image generated based on the correction data can be improved.

In some embodiments, the processing module 230 utilizes a second machine learning model to process the acquisition data based on the phase shift information and obtains the target image. In some embodiments, the second machine learning model includes but is not limited to, a Deep Neural Network (DNN), a Convolutional Neural Network (CNN) model, a Recurrent Neural Network (RNN), or the like. Specifically, inputs to the second machine learning model include the phase shift information and the acquisition data, and an output of the second machine learning model includes the target image.

In some embodiments, the second machine learning model may be obtained by training based on a plurality of second training samples with a second label. Specifically, the second training samples with the second label are input into an initial second machine learning model, and parameters of the initial second machine learning model are updated through training to obtain a trained second machine learning model. A second training sample of the second machine learning model includes sample phase shift information and sample acquisition data, and a second label includes a sample target image. The sample acquisition data may be historical medical data acquired by an imaging device, and the sample phase shift information may be determined manually based on the sample acquisition data or may be obtained by an algorithm (e.g., a pattern matching algorithm) or other manners with a relatively high degree of accuracy. The sample target image may be a gold standard image corresponding to historical medical data.

In some embodiments, the user selects to trigger the phase shift alignment function to process the acquisition data based on the phase shift information through the phase shift alignment function interface on the user interaction interface. The phase shift alignment function interface may include, but is not limited to, one or more of buttons, input boxes, selection boxes, sliders, menus, or the like, or any combination thereof.

Specifically, the processing module 230 obtains a second user request. In some embodiments, the second user request indicates a user selects to trigger a processing acceleration to convert the acquisition data into a minimal quantized signal through a processing acceleration function interface on a user interface. For example, as shown in FIG. 13, the user triggers the phase shift alignment function by clicking on the phase shift alignment button 1330 (i.e., the phase shift alignment function interface) on the user interaction interface 1300.

In some embodiments, the second user request indicates a user selects to trigger a processing acceleration to set one or more parameters of the processing acceleration through a processing acceleration function interface on a user interface. For example, as shown in FIG. 13, the user sets the parameters (e.g., an alignment manner is alignment between inter-frame data among acquisition data acquired at different times and/or alignment between data of different channels among acquisition data acquired at a same time) of the phase shift alignment function through a selection box combination 1340 (i.e., the phase shift alignment function interface) on the user interaction interface 1300. Furthermore, in response to the second user request, the processing module 230 performs the processing acceleration to convert the acquisition data into the minimal quantized signal, and performs the phase shift detection on the minimal quantized signal to obtain the phase shift information.

It should be noted that the description of the process 300 is provided for illustrative purposes only and is not intended to limit the scope of the present disclosure. For those of ordinary skill in the art, a wide variety of variations and modifications may be made in accordance with the description in the present disclosure. However, these changes and modifications do not depart from the scope of the present disclosure. In some embodiments, the above process may include one or more additional operations, or may omit one or more of the above operations.

In some embodiments, a determination module determines a movement speed of a region of interest in the target object based on the phase shift information. Merely by way of example, the determination module determines the movement speed of the region of interest in the target object based on phase shift information and time intervals of data sets corresponding to different acquisition times. For example, an acquisition time Ta corresponds to a phase shift map a, and an acquisition time Tb corresponds to a phase shift map b, a difference between the phase shift map a and the phase shift map b is calculated, a phase shift $\delta ab$ of the region of interest in the target object between the acquisition time Ta and the acquisition time Tb is determined, and a movement speed of the region of interest in the target object may be obtained based on $\delta ab/(Ta-Tb)$. Furthermore, the determination module generates a medical image representing the movement speed based on the movement speed of the region of interest in the target object and the acquisition data. For example, the medical image is a pulsed Doppler ultrasound image, a color blood flow ultrasound image, and/or a power Doppler ultrasound image.

In some embodiments, determining the movement speed of the region of interest in the target object based on the phase shift information enables acquisition of a pulsed Doppler ultrasound image, a color flow ultrasound image and/or a power Doppler ultrasound image based on acquisition data of a conventional ultrasound system without the need for additional scanning, thereby improving overall imaging efficiency and utilization of the acquisition data.

FIG. 4 is a flowchart illustrating an exemplary process for using a pattern matching algorithm to perform phase shift detection according to some embodiments of the present disclosure. In some embodiments, a process 400 is performed by the system 100 for data processing (e.g., the processing device 120) or the system for data processing 200 (e.g., the detection module 220). For example, the process 400 is stored in a storage device in the form of a program or instructions, and the process 400 is implemented when the system 100 for data processing (e.g., the processing device 120) or the system 200 for data processing (e.g., the detection module 220) executes the instructions. A schematic diagram of operations of the process 400 presented below is illustrative. In some embodiments, one or more additional operations not described and/or one or more operations not discussed may be utilized to complete the process. Additionally, the order of the operations of process 400 illustrated in FIG. 4 and described below is not limiting. As illustrated in FIG. 4, the process 400 may include one or more of the following operations.

In 410, a matching window and a searching window may be determined.

The matching window may limit information for matching a reference set and a target set. In some embodiments, a matching window for a reference set and a target set corresponding to an acquisition time may be two-dimensional, including a length in an axial direction and a width in a transverse direction. The length in the axial direction and the width in the transverse direction correspond to a Z-axis direction and an X-axis direction, respectively. In some embodiments, a matching window for a reference set and a target set corresponding to an acquisition channel may be one-dimensional, corresponding to a width in a transverse direction.

A length of the matching window in the axial direction may be a matching duration. In some embodiments, the length of the matching window in the axial direction is related to a center frequency of a signal received by the imaging device 110 (e.g., a probe). The smaller the center frequency of the signal received by the probe is, the longer the corresponding matching duration (i.e., the length of the matching window in the axial direction) is.

Specifically, the detection module 220 may determine a period of the center frequency based on the center frequency of the signal received by the probe. In some embodiments, the detection module 220 determines the period of the center frequency based on a center frequency of a signal received by a transducer. For example, if the center frequency of the signal received by the transducer fc is 2 MHz, then the period of the center frequency Tc=1/fc is 0.5 ms.

Further, the detection module 220 may determine the length of the matching window in the axial direction based on the period of the center frequency and a matching period. For example, if the period of the center frequency is 0.5 ms and the matching period is 2 periods of center frequency, a time length of the matching window in the axial direction is 1 ms.

In some embodiments, the matching period may be manually set. In some embodiments, the matching period may be determined through a neural network model. The neural network model may be obtained through training with training samples. A training sample includes sample acquisition data, and a training label includes a sample matching period. The sample matching period may be determined as follows: based on different matching periods, phase shift detection is performed on acquisition data to obtain a plurality of pieces of phase shift information, correspondingly. Then, phase correction is performed on the acquisition data based on the plurality of pieces of phase shift information to obtain a plurality of pieces of corresponding correction data. A plurality of medical images are generated based on the plurality of pieces of corresponding correction data, and a matching period corresponding to a medical image with the best quality is designated as the sample matching period. In some embodiments, the detection module 220 determines the matching period based on system arithmetic and/or accuracy requirements. The higher the system arithmetic and/or the higher the accuracy requirement, the higher the matching period may be set.

A width of the matching window in the transverse direction may be a count of match channels. For example, 1 channel, 3 channels, 5 channels. In some embodiments, the detection module 220 determines the width of the matching window in the transverse direction based on system arithmetic and/or accuracy requirements. The higher the system arithmetic and/or the higher the accuracy requirement, the higher the count of match channels may be set. For example, taking a resolution of the matching window in the transverse direction as an example, transverse resolution=wavelength of acquisition data×count of match channels. Therefore, the higher the requirement for transverse resolution, the higher the count of match channels.

The searching window may limit a match scope of the reference set and the target set. The searching window is larger than or equal to the matching window. In some embodiments, a dimension of the searching window corresponds to a dimension of the matching window.

The searching window is related to a maximum inter-frame displacement of a target object. In some embodiments, the detection module 220 determines a size of the searching window based on a maximum inter-frame displacement of a target object. Specifically, the detection module 220 may determine a maximum inter-frame displacement of the target object based on an estimated movement of the target object. Merely by way of example, the detection module 220 determines the maximum inter-frame displacement of the target object based on the estimated displacement speed of the target object and a repetition frequency of a pulse signal, e.g., the repetition frequency of the pulse signal is 10KHz, and the maximum displacement speed of the target object is v=5m/s, then the maximum inter-frame displacement of the target object is the maximum displacement speed of the target object (5 m/s) divided by the repetition frequency of the pulse signal (10 kHz), which equals to 0.5 mm. As another example, the detection module 220 tracks and predicts a displacement movement of the target object through manners such as Kalman filtering, to determine the maximum inter-frame displacement of the target object. Further, the detection module 220 may determine a size of a corresponding searching window based on the maximum inter-frame displacement of the target object. For example, the size of the searching window may be determined to be a region that can cover 0.5 mm of the maximum inter-frame displacement of the target object.

The searching window is negatively related to a correlation coefficient between the reference set and the target set. In some embodiments, the detection module 220 determines the size of the searching window based on a correlation coefficient between the reference set and the target set. The higher the correlation coefficient is, the smaller the searching window is. In some embodiments, the detection module 220 determines a corresponding correlation coefficient based on a distance between the reference set and the target set. In some embodiments, the distance includes but is not limited to a Euclidean distance, a Manhattan distance, a Chebyshev distance, a Minkowski distance, a Mars distance, a pinched cosine distance, or the like.

In 420, a phase shift coefficient between the target set and the reference set may be determined by using the pattern matching algorithm based on the matching window and the searching window to obtain phase shift information.

The phase shift coefficient may be a coefficient indicating a matching degree between reference search data corresponding to a searching window in the reference set and target search data corresponding to a searching window in the target set.

The target search data and the reference search data may be data used for matching in the target set and the reference set, respectively. In some embodiments, the detection module 220 moves the searching window based on a search step size to obtain at least a subset of target search data or a subset of reference search data in the target set or the reference set. In some embodiments, the detection module 220 determines the search step size based on a size of a data set, system arithmetic, and/or accuracy requirements. The smaller the data set, the higher the system arithmetic, and/or the higher the accuracy requirement, the smaller the search step may be set.

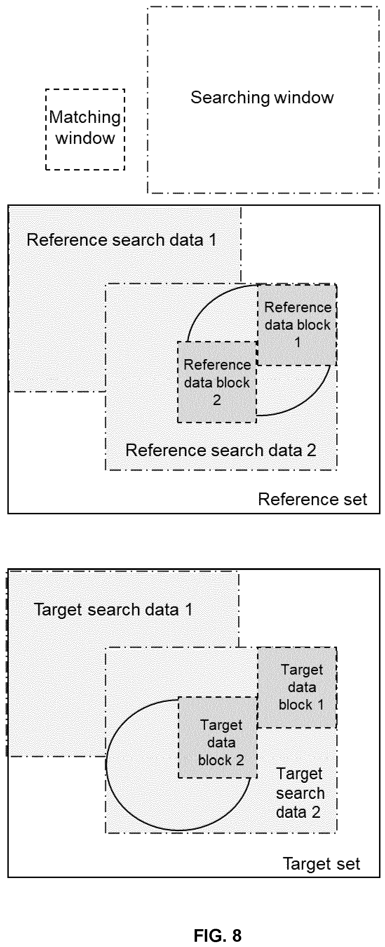

For example, FIG. 8 is a schematic diagram illustrating phase shift detection performed on a reference set and a target set based on a matching window and a searching window according to some embodiments of the present disclosure. As shown in FIG. 8, the detection module 220 moves a searching window in a reference set from a position 1 to position 2 based on a search step size, and obtains reference search data 1, reference search data 2, etc., corresponding to the searching window. The detection module 220 moves a searching window in a target set from a position 1 to a position 2 based on a search step size, and obtains target search data 1, target search data 2, etc., corresponding to the searching window. Phase shift coefficients may include phase shifts $R^{1,1}$, $R^{1,2}$ between the reference search data 1 and the target search data 1 and the target search data 2, respectively, and phase shift coefficients $R^{2,1}$, $R^{2,2}$ between the reference search data 2 and the target search data 1 and the target search data 2, respectively.

In some embodiments, the detection module 220 determines a phase shift coefficient between target search data and reference search data based on a plurality of target data blocks in the target search data and a plurality of corresponding reference data blocks in the reference search data. The target data block and the reference data block may be the smallest units of data used for matching. In some embodiments, the detection module 220 moves the matching window based on a move step size in a searching window of a target set or a reference set to obtain at least one target data block or reference data block. In some embodiments, the detection module 220 determines the move step size based on a size of a searching window, system arithmetic, and/or accuracy requirements. The smaller the searching window, the higher the system arithmetic, and/or the higher the accuracy requirement, the smaller the move step size may be set.

For example, as shown in FIG. 8, the detection module 220 moves a matching window based on a matching step size in the reference search data 2 to obtain a reference data block 1, a reference data block 2, etc., corresponding to the matching window; and in the target search data 2, the detection module 220 moves a matching window based on a matching step size to obtain a target data block 1, a target data block 2, etc., corresponding to the matching window.

In some embodiments, the phase shift coefficient includes at least one of a normalized cross correlation coefficient, a sum of square difference coefficient, or a sum of absolute differences coefficient.

The normalized cross correlation (NCC) coefficient may be a parameter used to represent a correlation between data. The normalized cross correlation coefficient takes values in a range -1 to 1, where the closer to -1 means the more negatively correlated the data is, the closer to 1 means the more positively correlated the data is, and the closer to 0 means the more uncorrelated the data is.

Merely by way of example, taking a two-dimensional matching window and a searching window as an example, the detection module 220 determines a normalized cross correlation coefficient between reference search data corresponding to a certain searching window and target search data corresponding to each searching window based on a Equation (1).

$$R_{NCC}(m,n) = \frac{\sum_{i,j=1,1}^{I,J}(F_c(\mathrm{i,j})*F_r(i,j))}{\sqrt{\sum_{i,j=1,1}^{I,J}F_r(\mathrm{i,j})^2}\sum_{i,j=1,1}^{I,J}F_c(\mathrm{i,j})^2}, \tag{1}$$

where $R_{NCC}(m, n)$ denotes a normalized cross correlation coefficient between target search data corresponding to a searching window at a position $(m, n)$ and reference search data corresponding to a certain searching window, $F_c(i, j)$ denotes a pixel value of a reference data block corresponding to a matching window in the i-th column and the j-th row of a searching window in reference search data, Fr(i,j) denotes a pixel value of a target data block corresponding to a matching window in the i-th column and the j-th row of a searching window in target search data, and $I, J$ indicate that each searching window may include I columns and J rows of matching windows.

The sum of square distance (SSD) coefficient may be a parameter used to indicate a variation degree between data. A range of a value of the sum of square distance coefficient is greater than or equal to 0, where a larger value indicates a greater variation between data.

Merely by way of example, taking a two-dimensional matching window and a searching window as an example, the detection module 220 calculates a sum of square distance coefficient between reference search data corresponding to a certain searching window and target search data corresponding to each searching window based on a Equation (2).

$$R_{SSD}(m,n) = \sum_{i,j=1,1}^{I,J}(F_c(i,j) - F_r(i,j))^2, \tag{2}$$

where $R_{SSD}(m, n)$ denotes a sum of square distance coefficient between target search data corresponding to s searching window at s position $(m, n)$ and reference search data corresponding to a certain searching window, $F_c(i, j)$ denotes a pixel value of reference data block corresponding to a matching window in the i-th column and the j-th row of a searching window in reference search data, $F_r(i, j)$ denotes a pixel value of a target data block corresponding to a matching window in the i-th column and the j-th row of a searching window in target search data, and $I, J$ indicate that each searching window may include I columns and J rows of matching windows.

The sum of absolute differences (SAD) coefficient may be a parameter used to indicate a variation degree between data. A range of a value of the sum of absolute differences coefficient is greater than or equal to 0, wherein a larger value indicates a greater variation between data.

Merely by way of example, taking a two-dimensional matching window and a searching window as an example, the detection module 220 may calculate a sum of absolute difference coefficient between reference search data corresponding to a certain searching window and target search data corresponding to each searching window based on an Equation (3).

$$R_{SAD}(m,n) = \sum_{i,j=m,n}^{I-m,J-n}|F_c(\mathrm{i,j}) - F_r(i,j)|, \tag{3}$$

where $R_{SAD}(m, n)$ denotes a sum of absolute differences coefficient between target search data corresponding to a searching window at a position $(m, n)$ and reference search data corresponding to a certain searching window, $F_c(i, j)$ denotes a pixel value of a reference data block corresponding to a matching window in the i-th column and the j-th row of a searching window in reference search data, $F_r(i, j)$ denotes a pixel value of a target data block corresponding to a matching window in the i-th column and the j-th row of a searching window in target search data, and $I, J$ indicate that each searching window may include I columns and J rows of matching windows.

In some embodiments, the phase shift coefficient includes a bitwise cross-correlation data stream between the target set and the reference set. The bitwise cross-correlation (BCC) data stream may be a coefficient indicating a matching degree between reference search data corresponding to a searching window in a reference set and target search data corresponding to a searching window in a target set on each channel. A detailed description of obtaining the bitwise cross-correlation data stream can be found in FIG. 5 and will not be repeated herein.

The pattern matching algorithm may be an algorithm that determines a displacement between a reference set and a target set based on the phase shift coefficient. In some embodiments, the detection module 220 obtains an extreme value among phase shift coefficients between reference search data corresponding to each searching window in a reference set and a plurality of pieces of target search data corresponding to a plurality of searching windows in a target set, and determines a position of a searching window in the target set corresponding to the extreme value as a position in a phase shift map.

Merely by way of example, in the case of the normalized cross correlation coefficient, for normalized cross correlation coefficients -0.2, 0, 0.1, ..., between reference search data 1 corresponding to a searching window at a position 1 in a reference set, and target search data 1, target search data 2, target search data 3, ... in a target set, an extreme value among the coefficients is -0.2, which is the minimal value, then position coordinates $(1, 1)$ of a searching window $R_{SAD}(1, 1)$ of the target search data 1 corresponding to -0.2 may be determined as position coordinates corresponding to a position 1 in a phase shift map.

As another example, in the case of the sum of square distance coefficient, for sum of square distance coefficients of 3, 6, 1, ... between reference search data 2 corresponding to a searching window at a position 2 in a reference set and target search data 1, target search data 2, target search data 3, ... in a target set, an extreme value among the coefficients is 6, which is the maximum value, then position coordinates $(2, 3)$ of a searching window $R_{SSD}(2, 3)$ of the target search data 2 corresponding to 6 may be determined as position coordinates corresponding to a position 2 in a phase shift map. Similarly, the detection module 220 may obtain a position of each searching window in the reference set that corresponds to a searching window in the target set, thereby obtaining the phase shift map.

In some embodiments, the detection module 220 obtains the phase shift information based on a bitwise cross-correlation data stream of each channel between the target set and the reference set. Specifically, after obtaining the bitwise cross-correlation data stream of the each channel, the detection module 220 may obtain a corresponding phase shift map of the each channel.

In some embodiments of the present disclosure, based on the matching window and the searching window, the pattern matching algorithm is utilized to determine the phase shift coefficient between the target set and the reference set, which can improve the accuracy and efficiency of the matching between the target set and the reference set, thereby obtaining accurate phase shift information.

It should be noted that the description of the process 400 is provided for illustrative purposes only and is not intended to limit the scope of the present disclosure. For those of ordinary skill in the art, a wide variety of variations and modifications may be made in accordance with the description in the present disclosure. However, these changes and modifications do not depart from the scope of the present disclosure. In some embodiments, the above process may include one or more additional operations, or may omit one or more of the above operations.

FIG. 5 is a flowchart illustrating an exemplary process for obtaining a bitwise cross-correlation data stream according to some embodiments of the present disclosure. In some embodiments, a process 500 is performed by the system 100 for data processing (e.g., the processing device 120) or the system 200 for data processing (e.g., the detection module 220). For example, the process 500 may be stored in a storage device in the form of a program or instructions, and the process 500 may be implemented when the system 100 for data processing (e.g., the processing device 120) or the system 200 for data processing (e.g., the detection module 220) executes the instructions. A schematic diagram of operations of the process 500 presented below is illustrative. In some embodiments, the process may be accomplished utilizing one or more additional operations that are not described and/or one or more operations that are not discussed. Additionally, the order of the operations of process 500 illustrated in FIG. 5 and described below is not limiting. As illustrated in FIG. 5, the process 500 may include one or more of the following operations.

In 510, acquisition data may be converted to a minimal quantized signal (e.g., a phase shift event bit stream).

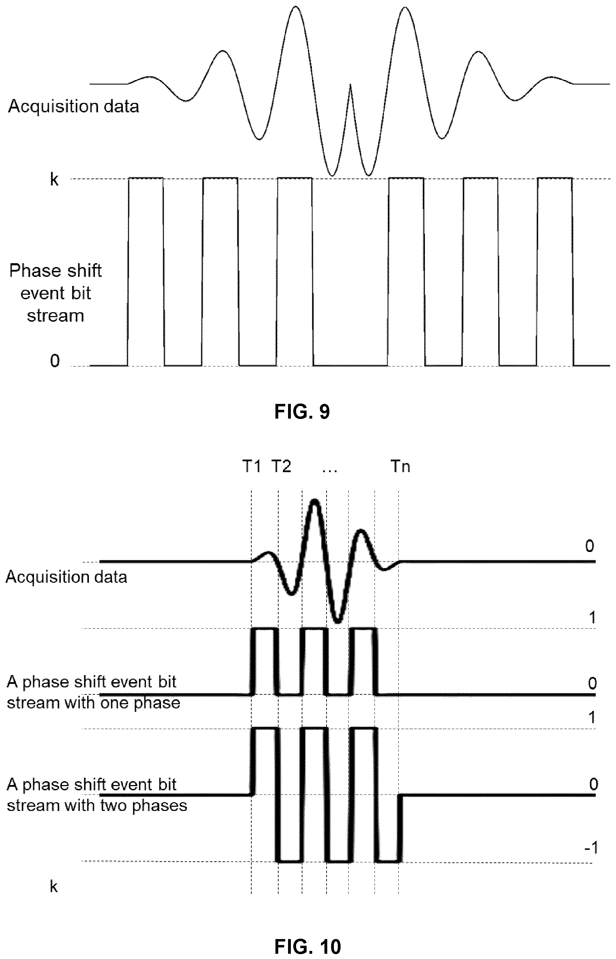

The minimal quantized signal is the smallest quantifiable signal. The minimal quantized signal may be or include a phase shift event bit stream (PSEBS), which is a data stream with only phase shift information and time information. FIG. 9 is a schematic diagram illustrating an exemplary phase shift event bit stream corresponding to acquisition data according to some embodiments of the present disclosure. As shown in FIG. 9, the acquisition data represents a change in amplitude of data over time, and a corresponding phase shift event bit stream only represents whether or not the data has been subjected to a phase shift over time, wherein a phase shift is indicated by a value k and no phase shift is indicated by a value 0.

In some embodiments, the detection module 220 converts an amplitude of the acquisition data into a phase shift event bit stream of at least one phase based on a conversion threshold. FIG. 10 is a schematic diagram illustrating two exemplary manners of converting acquisition data to a phase shift event bit stream according to some embodiments of the present disclosure. As shown in FIG. 10, the acquisition data is converted into a phase shift event bit stream with one phase based on a conversion threshold of 0, i.e., amplitudes greater than 0 in the acquisition data are converted to phase 1, while other amplitudes are considered to have no phase (represented as 0). Alternatively, the acquisition data is converted into a phase shift event bit stream with two phases based on the conversion threshold of 0, i.e., amplitudes greater than 0 in the acquisition data are converted to phase 1, while amplitudes less than 0 are converted to phase -1.

In some embodiments, a user may select to trigger a PSEBS processing acceleration function to convert the acquisition data into the phase shift event bit stream through a PSEBS processing acceleration function interface on a user interaction interface. The PSEBS processing acceleration function interface may include but is not limited to, one or more buttons, input boxes, selection boxes, sliders, menus, or the like, or any combination thereof.

Specifically, the detection module 220 obtains a third user request. In some embodiments, the third user request indicates a user selects to trigger a phase alignment to process the acquisition data based on the phase shift information through a phase alignment function interface on a user interface. For example, as shown in FIG. 13, a user may trigger a PSEBS processing acceleration function by clicking on a PSEBS processing acceleration button 1350 (i.e., a PSEBS processing acceleration function interface) on the user interaction interface 1300.

In some embodiments, the third user request indicates a user selects to trigger a phase alignment to set one or more parameters of the phase alignment through a phase alignment function interface on a user interface. For example, the user sets parameters (e.g., a conversion threshold) of the PSEBS processing acceleration function through an input box (not shown in the figure) on the user interaction interface 1300. Furthermore, in response to the third user request, the detection module 220 processes the acquisition data based on the phase shift information.

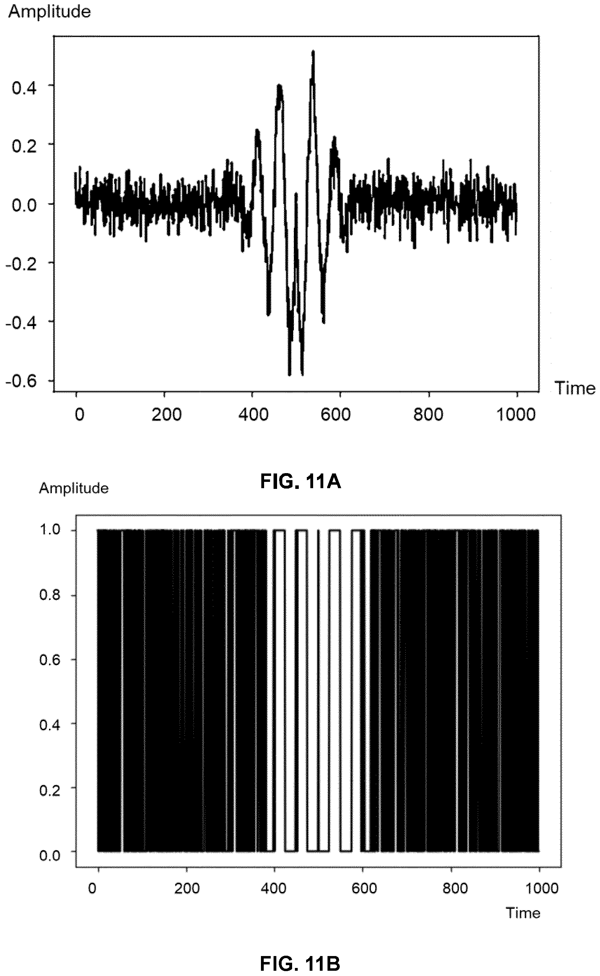

In some embodiments, the detection module 220 preprocesses the acquisition data before converting the acquisition data into the phase shift event bit stream. In some embodiments, a pre-processing includes a noise-reduction processing. For example, FIGs. 11A and 11B are schematic diagrams illustrating acquisition data and a corresponding phase shift event bit stream according to some embodiments of the present disclosure. As shown in FIG. 11A, the data includes noises, which may cause a phase shift event bit stream to generate a high-frequency bounce as shown in FIG. 11B. Therefore, a noise-reduction processing is required to be performed on the acquisition data. Merely by way of example, the detection module 220 filters the acquisition data using a linear phase low-pass filter, such as a finite impulse response (FIR) filter, to achieve the noise-reduction processing.

In some embodiments, the pre-processing includes direct current (DC) removal. DC removal refers to the removal of direct current shifts present in the acquisition data. The presence of a direct current shift in an acquisition signal can cause the phase shift event bit stream to have long periods of "1" or "0". Merely by way of example, the detection module 220 performs the DC removal on the acquisition data by simple decimation accumulation or linear phase high-pass filtering (e.g., using cascaded low-pass and high-pass filters, band-pass filters).

In some embodiments, the pre-processing includes upsampling. In order to improve the phase accuracy of the phase shift event bit stream, data of one or more channels may be upsampled. Merely by way of example, the detection module 220 upsamples the acquisition data using a linear phase interpolation filter. The linear phase interpolation filter may enable both noise reduction and DC removal of the acquisition data.

In some embodiments, the pre-processing includes zero cross detection. Specifically, the detection module 220 may detect a moment at which a signal waveform of the acquisition data crosses a zero point. For example, as shown in FIG. 10, the detection module 220 detects moments T1, T2, ..., Tn at which the signal waveform of the acquisition data crosses the zero point. The zero cross detection may provide phase information for converting the acquisition data to the phase shift event bit stream.

In some embodiments, the detection module 220 post-processes the phase shift event bit stream to eliminate a high-frequency bounce. Merely by way of example, the detection module 220 counts a time interval corresponding to a phase change from high to low or a phase change from low to high of the phase shift event bit stream, and if the time interval is less than a preset first threshold or greater than a preset second threshold, "1" or "0" value is not changed, otherwise, the "1" or "0" value is changed. The first threshold and the second threshold may be determined based on a bandwidth of a signal of interest in the acquisition data.

In 520, phase shift detection may be performed on the minimal quantized signal to obtain a bitwise cross-correlation data stream of each channel between a target set and a reference set.

As can be seen from the foregoing, the bitwise cross-correlation (BCC) data stream may include a coefficient indicating a matching degree between reference search data corresponding to a searching window in the reference set and target search data corresponding to a searching window in the target set on each channel.

Specifically, in some embodiments, the detection module 220 selects a region of interest. The region of interest may be the entire data set or a portion of a region in a data set. Further, in some embodiments, the detection module 220 determines a bitwise cross-correlation data stream between a minimal quantized signal (e.g., a phase shift event bit stream) of each channel in a region of interest in the reference set and a minimal quantized signal (e.g., a phase shift event bit stream) of each channel in the target set based on a matching window and a searching window using a pattern matching algorithm.

A detailed description of the pattern matching algorithm can be found in FIG. 4 and its related descriptions, and will not be repeated here.

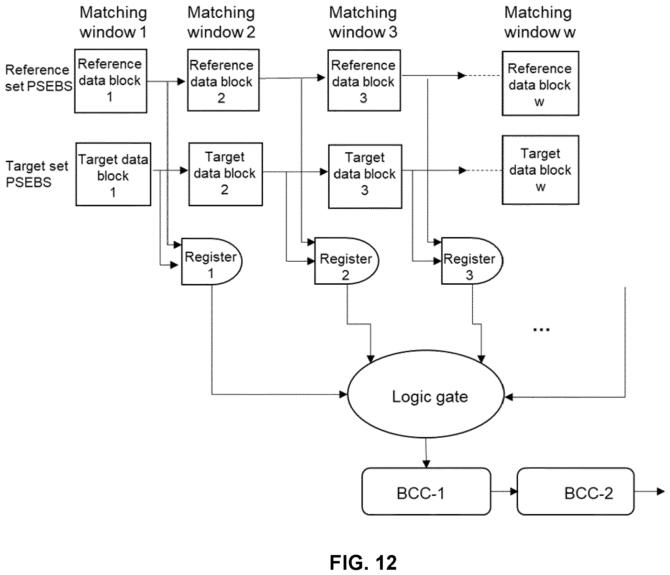

In some embodiments, the detection module 220 implements the pattern matching algorithm for PSEBS based on logic gates. FIG. 12 is a schematic diagram illustrating an exemplary operating principle of phase shift detection performed on a phase shift event bit stream according to some embodiments of the present disclosure. As shown in FIG. 12, the operating principle of the phase shift detection for the phase shift event bit stream may be regarded as a Finite Impulse Response (FIR) filter. The PSEBS of a reference set may be considered as coefficients of the FIR filter, while the PSEBS of a target set may be designated as input data for the FIR filter. Based on w matching windows in a searching window (not shown in the figure), w registers are used to obtain w reference data blocks on a channel 1 in the PSEBS of the reference set and w target data blocks on a channel 1 in the PSEBS of the target set, respectively, logic gates are then used to calculate BCC-1 on the channel 1 corresponding to the searching window (not shown in the figure). In a similar manner, the detection module 220 may further compute BCC-2, BCC-3, ..., on a channel 2.

In some embodiments, the logic gates may include, but are not limited to, an AND gate, an XOR gate, or the like. Merely by way of example, when the logic gate is a AND gate, the larger the bitwise cross-correlation data stream (equivalent to the NCC coefficient) corresponding to each channel, the better the match between a reference set and a target set. As another example, when the logic gate is an XOR gate, the smaller the bitwise cross-correlation data stream corresponding to each channel (equivalent to the SSD coefficient or the SAD coefficient), the better the match between a reference set and a target set.

In some embodiments of the present specification, converting the acquisition data into a minimal quantized signal that eliminates the magnitude information results in a significantly reduced amount of data in the minimal quantized signal (e.g., only one or two bits) while retaining the phase shift information, resulting in fewer subsequent calculations to compute the inter-correlation data stream and the phase shift information, and improving the computational efficiency of the system.

It should be noted that the description of the process 500 is provided for illustrative purposes only and is not intended to limit the scope of the present disclosure. For those of ordinary skill in the art, a wide variety of variations and modifications may be made in accordance with the description in the present disclosure. However, these changes and modifications do not depart from the scope of the present disclosure. In some embodiments, the above process may include one or more additional operations, or may omit one or more of the above operations.

Beneficial effects brought by embodiments of the present disclosure include, but are not limited to: (1) by performing the phase shift detection on the acquisition data to obtain the phase shift information, performing the phase correction on the acquisition data based on the phase shift information, and obtaining the correction data, such that the correction data can eliminate the effects of motion displacement between data sets corresponding to different frames (i.e., different acquisition times), the effects of delay processing due to different acquisition channels, and/or the effects of delay processing between the acquisition channels and the delay configuration between systems, thereby improving the signal-to-noise ratio of a medical image generated based on the correction data; (2) calculating the phase shift coefficients between the target set and the reference set based on the matching window and the searching window using the pattern matching algorithm can improve the accuracy and efficiency of matching between the target set and the reference set, thereby acquiring accurate phase shift information; (3) converting the acquisition data into the minimal quantized signal greatly reduces the amount of data in the minimal quantized signal (e.g., only one or two bits) while retaining the phase shift information, thus reducing the amount of computation for subsequent calculation of the cross-correlation data stream and the phase shift information, and improving the computational efficiency of the system; (4) based on the phase shift information, phase alignment on inter-frame data among the acquisition data acquired at different times can eliminate motion artifacts in the medical images generated from multiple data sets corresponding to different acquisition times; and/or phase alignment on data of different channels among the acquisition data acquired at a same time can eliminate deviations caused by delay processing across multiple channels or misalignment in the delay mismatches between acquisition channels; (5) multi-frame beamforming based on the correction data to generate the composite image can be performed, which can eliminate the shift generated by delay processing of multiple channels or delay mismatches between acquisition channels; and/or generating the target image based on the correction data for single-frame image reconstruction can eliminate motion artifacts in medical images generated from multiple data sets corresponding to multiple acquisition times. It should be noted that the beneficial effects that may be produced by different embodiments are different, and the beneficial effects that may be produced in different embodiments may be any one or a combination of the foregoing, or any other beneficial effect that may be obtained.

The basic concepts have been described above, and it is apparent to those skilled in the art that the foregoing detailed disclosure is intended as an example only and does not constitute a limitation of the present disclosure. While not expressly stated herein, a person skilled in the art may make various modifications, improvements, and amendments to the present disclosure. Those types of modifications, improvements, and amendments are suggested in the present disclosure, so those types of modifications, improvements, and amendments remain within the spirit and scope of the exemplary embodiments of the present disclosure.

Also, the present disclosure uses specific words to describe embodiments of the present disclosure. Such as "an embodiment", "one embodiment", and/or "some embodiments" means a feature, structure, or characteristic associated with at least one embodiment of the present disclosure. Accordingly, it should be emphasized and noted that "an embodiment" or "one embodiment" or "an alternative embodiment" in different places in the present disclosure do not necessarily refer to the same embodiment. In addition, certain features, structures, or characteristics of one or more embodiments of the present disclosure may be suitably combined.

Additionally, the order in which the elements and sequences are processed in the present disclosure, the use of numerical letters, or the use of other names is not intended to qualify the order of the processes and methods of the present disclosure, unless expressly stated in the claims. While some embodiments of the invention that are currently considered useful are discussed in the foregoing disclosure by way of various examples, it should be appreciated that such details serve only illustrative purposes, and that additional claims are not limited to the disclosed embodiments, rather, the claims are intended to cover all amendments and equivalent combinations that are consistent with the substance and scope of the embodiments of the present disclosure. For example, although the implementation of various components described above may be embodied in a hardware device, it may also be implemented as a software only solution, e.g., an installation on an existing server or mobile device.

Similarly, it should be noted that in order to simplify the presentation of the present disclosure, and thereby aid in the understanding of one or more embodiments of the invention, the foregoing descriptions of embodiments of the present disclosure sometimes set multiple features together in a single embodiment, accompanying drawings, or a description thereof. However, this method of disclosure does not imply that the objects of the present disclosure require more features than those mentioned in the claims. Rather, claimed subject matter may lie in less than all features of a single foregoing disclosed embodiment.

Some embodiments use numbers to describe the number of components and attributes, and it should be understood that such numbers used in the description of the embodiments are modified in some examples by the modifiers "about", "approximately", or "substantially". Unless otherwise noted, the terms "about," "approximately," or "substantially" indicate that a $\pm20\%$ variation in numbers is allowed. Correspondingly, in some embodiments, the numerical parameters used in the present disclosure and claims are approximations, which can change depending on the desired characteristics of individual embodiments. In some embodiments, the numerical parameters should take into account the specified number of valid digits and employ general place-keeping. While the numerical domains and parameters used to confirm the breadth of their ranges in some embodiments of the present disclosure are approximations, in specific embodiments, such values are set to be as precise as possible within a feasible range.

For each of the patents, patent applications, patent application disclosures, and other materials cited in the present disclosure, such as articles, books, specification sheets, publications, documents, etc., the entire contents of which are hereby incorporated herein by reference. Application history documents that are inconsistent with or conflict with the contents of the present disclosure are excluded, as are documents (currently or hereafter appended to the present disclosure) that limit the broadest scope of the claims of the present disclosure. It should be noted that in the event of any inconsistency or conflict between the descriptions, definitions, and/or use of terms in the materials appended to the present disclosure and the contents of the present disclosure, the descriptions, definitions, and/or use of terms in the present disclosure shall prevail.

Finally, it should be understood that the embodiments in the present disclosure are only used to illustrate the principles of the embodiments in the present disclosure. Other deformations may also fall within the scope of the present disclosure. As such, alternative configurations of embodiments of the present disclosure may be considered to be consistent with the teachings of the present disclosure as an example, not as a limitation. Correspondingly, the embodiments of the present disclosure are not limited to the embodiments expressly presented and described herein.

**Claims**

1. A method implemented on at least one machine each of which has at least one processor and a storage device for data processing, the method comprising:

   obtaining acquisition data of a target object;
   obtaining phase shift information by performing phase shift detection on the acquisition data; and
   generating a target image by processing the acquisition data based on the phase shift information.

2. The method of claim 1, wherein the generating a target image by processing the acquisition data based on the phase shift information includes:

   obtaining correction data by performing phase correction on the acquisition data based on the phase shift information; and

generating the target image based on the correction data.

3.  The method of claim 1 or 2, wherein the acquisition data includes ultrasound imaging data.

4.  The method of any one of claims 1-3, wherein the acquisition data includes a plurality of data sets, the plurality of data sets including a reference set and at least one target set.

5.  The method of claim 4, wherein the performing phase shift detection on the acquisition data includes:

    determining a matching window and a searching window; and
    obtaining the phase shift information by determining, using a pattern matching algorithm based on the matching window and the searching window, one or more phase shift coefficients between the at least one target set and the reference set.

6.  The method of claim 5, wherein the one or more phase shift coefficients include at least one of a normalized cross correlation coefficient, a sum of square difference coefficient, or a sum of absolute difference coefficient.

7.  The method of claim 5 or 6, wherein a length of the matching window along a timeline of the acquisition data is related to a center frequency of the acquisition data.

8.  The method of any one of claims 5-7, wherein the one or more phase shift coefficients include at least one bitwise cross-correlation data stream between the at least one target set and the reference set; and the obtaining phase shift information by performing phase shift detection on the acquisition data includes:

    converting the acquisition data into a minimal quantized signal; and
    performing the phase shift detection on the minimal quantized signal to obtain a bitwise cross-correlation data stream of each channel between the at least one target set and the reference set.

9.  The method of claim 8, wherein before converting the acquisition data into the minimal quantized signal, the method further comprising:

    upsampling the acquisition data; and/or
    performing zero cross detection on the acquisition data.

10. The method of any one of claims 5-9,

    wherein the matching window is two-dimensional, including a length in an axial direction and a width in a transverse direction;
    wherein the determining a matching window and a searching window includes:

        determining a period of a center frequency based on the center frequency of the acquisition data;
        determining a length of the matching window in the axial direction based on the period of the center frequency.

11. The method of any one of claims 5-10, wherein

    the searching window is negatively related to a correlation coefficient between the reference set and the at least one target set; and/or
    the searching window is related to a maximum inter-frame displacement of the target object.

12. The method of claim 2, wherein the obtaining correction data by performing phase correction on the acquisition data based on the phase shift information includes:

    performing, based on the phase shift information, phase alignment on inter-frame data among the acquisition data acquired at different times; and/or
    performing, based on the phase shift information, phase alignment on data of different channels among the acquisition data acquired at a same time.

13. The method of claim 2 or 12, wherein the generating the target image based on the correction data includes:

generating a composite image by performing multi-frame beamforming based on the correction data; and/or generating the target image by performing single-frame image reconstruction based on the correction data.

14. A system, comprising:

at least one storage medium storing a set of instructions; and
at least one processor configured to communicate with the at least one storage medium, wherein when executing the set of instructions, the at least one processor is directed to cause the system to perform operations including:

obtaining acquisition data of a target object;
obtaining phase shift information by performing phase shift detection on the acquisition data; and
generating a target image by processing the acquisition data based on the phase shift information.

15. A non-transitory computer readable medium, comprising at least one set of instructions, wherein when executed by at least one processor of a computer device, the at least one set of instructions directs the at least one processor to perform operations including:

obtaining acquisition data of a target object;
obtaining phase shift information by performing phase shift detection on the acquisition data; and
generating a target image by processing the acquisition data based on the phase shift information.

100

FIG. 1

FIG. 2

300

Obtaining acquisition data of a target object
310

Performing phase shift detection on the acquisition data to obtain phase shift information
320

Processing the acquisition data based on the phase shift information to generate a target image
330

FIG. 3

**400**

Determining a matching window and a searching window
410

Determining a phase shift coefficient between a target set and a reference set using a pattern matching algorithm based on the matching window and the searching window to obtain phase shift information
420

**FIG. 4**

**500**

Converting acquisition data into a minimal quantized signal
510

Performing phase shift detection on the minimal quantized signal to obtain a bitwise cross-correlation data stream of each channel between a target set and a reference set
520

**FIG. 5**

$y_a$

$x_a$

Acquisition time Ta

$y_b$

$x_b$

Acquisition time Tb

FIG. 6A

$h_c$

$I_c$

Acquisition time Tc

$h_d$

$I_d$

Acquisition time Td

FIG. 6B

**FIG. 7A**

**FIG. 7B**

Matching window

Searching window

Reference search data 1

Reference data block 1

Reference data block 2

Reference search data 2

Reference set

Target search data 1

Target data block 1

Target data block 2

Target search data 2

Target set

FIG. 8

Acquisition data

Phase shift event bit stream

k

0

**FIG. 9**

T1 T2 ... Tn

Acquisition data

A phase shift event bit stream with one phase

A phase shift event bit stream with two phases

k

**FIG. 10**

FIG. 11A

FIG. 11B

FIG. 12

1300

1320

Search step size_____

Matching period _____

Accuracy requirements__

Alignment manner:

☐ Phase alignment on inter-frame data among acquisition data acquired at different times

☐ Phase alignment on data of different channels among acquisition data acquired at a same time

1340

Phase shift detection 1310

Phase alignment 1330

PSEBS processing acceleration 1350

FIG. 13

Europäisches
Patentamt
European
Patent Office
Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KARAMAN M ET AL: "VLSI CIRCUITS FOR ADAPTIVE DIGITAL BEAMFORMING IN ULTRASOUND IMAGING", IEEE TRANSACTIONS ON MEDICAL IMAGING, IEEE, USA, vol. 12, no. 4, 1 December 1993 (1993-12-01), pages 711-720, XP000447018, ISSN: 0278-0062, DOI: 10.1109/42.251122 * abstract; figure 4 * * chapter I * * chapter II * * chapter III * * chapter V * * chapter VI, last paragraph * ----- | 1-10, 12-15 | INV. G01S7/52 G01S15/89 A61B8/00 |
| X | EP 3 424 434 A1 (KONINKLIJKE PHILIPS NV [NL]) 9 January 2019 (2019-01-09) * abstract; figure 4 * * paragraphs [0006], [0007], [0037] * * paragraphs [0050], [0051], [0068] * * paragraphs [0082], [0083], [0086] * * paragraphs [0088], [0093], [0096] * * paragraphs [0097], [0169], [0170] * ----- | 1-7, 10-15 | |
| | | | **TECHNICAL FIELDS SEARCHED (IPC)** G01S A61B |

The present search report has been drawn up for all claims

| Place of search | Date of completion of the search | Examiner |
|---|---|---|
| Munich | 6 May 2025 | Knoll, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 22 3278

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2025

| Patent document cited in search report | | Publication date | Patent family member(s) | | Publication date |
|---|---|---|---|---|---|
| EP 3424434 | A1 | 09-01-2019 | CN | 110868938 A | 06-03-2020 |
| | | | EP | 3424434 A1 | 09-01-2019 |
| | | | EP | 3648673 A1 | 13-05-2020 |
| | | | JP | 7130008 B2 | 02-09-2022 |
| | | | JP | 2020526268 A | 31-08-2020 |
| | | | US | 2020229797 A1 | 23-07-2020 |
| | | | WO | 2019008187 A1 | 10-01-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311814636